# EUROPÄISCHE PATENTSCHRIFT

(11) **EP 1 742 709 B1**
(45) Veröffentlichungstag und Bekanntmachung des Hinweises auf die Patenterteilung: **17.06.2009**
(21) Anmeldenummer: 04765997.4
(22) Anmeldetag: 20.10.2004
(51) Int. Cl.: A61Q 5/10, A61K 8/42, A61K 8/39, A61K 8/34, A61Q 5/06

(54) **FÄRBEMITTEL MIT PERLGLANZ FÜR KERATINFASERN**
COLORING AGENT HAVING A PEARLY LUSTER FOR KERATIN FIBERS
PRODUIT COLORANT A BRILLANCE NACREE POUR FIBRES DE KERATINE

(30) Priorität: 05.02.2004 DE 102004005769
(43) Veröffentlichungstag der Anmeldung: 17.01.2007
(73) Patentinhaber: The Procter and Gamble Company, Cincinnati, Ohio 45202 (US)
(72) Erfinder: DÖHLING, Annelie, 64839 Münster (DE); KREHER, Helga, 64839 Münster (DE); LAUSCHER, Dirk, 64297 Darmstadt (DE)
(74) Vertreter: Bockhorst, Matthias
(86) Internationale Anmeldenummer: PCT/EP2004/011854
(87) Internationale Veröffentlichungsnummer: WO 2005/074871

(56) Entgegenhaltungen:
- EP-A- 1 293 192
- EP-A- 1 428 509
- WO-A-20/04019895
- DE-A- 3 506 543
- DE-A- 19 544 655
- DE-A- 19 847 224
- DE-C- 4 331 136
- DE-C- 19 825 133
- US-A1- 2003 074 747

## Beschreibung

Gegenstand der Erfindung sind perlmuttartig glänzende Mittel zum Färben von Keratinfasern, insbesondere menschlichen Haaren, mit einem Gehalt an direktziehenden und/oder oxidativen Farbstoffen und einer speziellen Kombination aus Fettalkoholen, Fettsäurealkanolamiden und Fettalkoholalkoxylaten und/oder Fettsäurealkoxylaten, sowie die Verwendung der vorgenannten Kombination zur Erzeugung eines stabilen Perlglanzes in Haarfärbemitteln.

Färbende Präparate liegen üblicherweise in Form von wässrigen -vorzugsweise verdickten- Lösungen oder Emulsionen vor und enthalten neben Farbstoffen beispielsweise Fettalkohole und/oder andere Ölkomponenten, Emulgatoren und Tenside, sowie gegebenenfalls Alkohole. Oxidationsfärbemittel bestehen in der Regel aus zwei Komponenten, (i) der die Farbstoffe enthaltenden Farbstoffträgermasse und (ii) der Oxidationsmittelzubereitung, die kurz vor dem Gebrauch miteinander vermischt und dann auf das zu färbende Haar aufgetragen werden. Liegen die färbenden Präparate als Emulsionen vor, so sind diese in der Regel stabile Cremes, die jedoch für die Erzeugung eines Perlmuttglanzeffektes den Zusatz von speziellen Perlglanzmitteln benötigen.

Aus der DE-A 38 34 142, EP-A 1 293 192, DE-C 43 31 136, DE-C 198 25 133, DE-A 195 44 655 und US-A 2003/074747 sind Haarfärbemittel bekannt, welche eine Vielzahl von Rohstoffen, u.a. auch Fettalkohole und Fettsäurealkanolamide sowie anionische und nichtionische Tenside enthalten können. Diese Mittel weisen jedoch keinen Perlglanz auf.

Es bestand daher die Aufgabe, eine Farbmasse zu entwickeln, die ohne den Zusatz von Perlglanzmitteln, alleine durch die Auswahl der Rohstoffe einen stabilen perlmuttartigen Charakter aufweist, der auch nach dem Vermischen mit der Oxidationsmittelzubereitung bestehen bleibt.

Es wurde nunmehr gefunden, dass durch Verwendung einer Kombination aus einem Fettalkohol, einem Fettsäurealkanolamid und einem Fettalkoholalkoxylat und/oder Fettsäurealkoxylat diese Aufgabe in hervorragender Weise gelöst wird.

Gegenstand der vorliegenden Erfindung ist daher eine Farbträgermasse, enthaltend oxidative und/oder nicht-oxidative ("direktziehende") Farbstoffe, dadurch gekennzeichnet, dass sie frei von monomeren quaternären Ammoniumverbindungen sowie anionischen und kationischen Emulgatoren und Tensiden ist und eine Kombination aus
(a) 6,1 bis 25 Gewichtsprozent mindestens eines Fettalkohols mit 14 bis 20 Kohlenstoffatomen,
(b) 1 bis 20 Gewichtsprozent mindestens eines Fettsäurealkanolamids und
(c) 1 bis 15 Gewichtsprozent mindestens eines Fettalkoholalkoxylats und/oder Fettsäurealkoxylats enthält,
wobei das Gewichtsverhältnis von Fettalkohol (a) zu Fettsäurealkanolamid (b) gleich 4:1 bis 1:3 und gleichzeitig das Gewichtsverhältnis von Fettalkohol (a) zu Alkoxylat (c) gleich 5:1 bis 1:2 ist.

Für die Ausbildung eines schönen Perlmuttglanzes wird ein Gewichtsverhältnis von Fettalkohol (a) zu Alkanolamid (b) in der Farbträgermasse von 3:1 bis 1:2 und insbesondere ein Gewichtsverhältnis von 2,5 :1 bis 1:1 bevorzugt. Das Gewichtsverhältnis von Fettalkohol (a) zu Alkoxylat (c) beträgt hierbei 4:1 bis 1:1,5 und insbesondere 3:1 bis 1:1.

Erfindungsgemäß geeignete langkettige Fettalkohole (a) mit 14 bis 20 Kohlenstoffatomen sind zum Beispiel Cetylakohol, Stearylalkohol, Myristylalkohol, Isooctylalkohol oder Isotridecylalkohol. Die Fettalkohole können in der erfindungsgemäßen Farbträgermasse sowohl einzeln als auch in Kombination miteinander eingesetzt werden.

Erfindungsgemäß geeignete Alkanolamide (b) sind insbesondere die N-Acylderivate des Monoethanolamins, wie zum Beispiel das Kokosnussfettsäuremonoethanolamid, oder die N-Acylderivate des Diethanolamins sowie die Monoethanolaminester oder Diethanolaminester. Die Alkanolamide können sowohl einzeln als auch in Kombination miteinander eingesetzt werden.

Erfindungsgemäß geeignete Alkoxylate (c) sind Fettalkoholpolyalkylenglykolether und/oder Fettsäurepolyalkylenglykolester mit 8 bis 30 Kohlenstoffatomen im Fettalkoholrest beziehungsweise Fettsäurerest und 2 bis 300 Alkylenglykoleinheiten im Polyalkylenglykolrest.

Bevorzugt sind Polyalkylenglykolalkoxylate des Ethylenglykols oder Propylenglykols sowie kombinierte Polyethylenglykolalkoxylate/ Polypropylenglykolalkoylate. Besonders bevorzugt sind Fettalkoholalkoxylate wie zum Beispiel Polyethylenglykolether des Stearylalkohols, beispielsweise Steareth-10, Ceteareth-25 oder Steareth-20.

Die Fettalkoholalkoxylate und Fettsäurealkoxylate können sowohl einzeln als auch in Kombination miteinander eingesetzt werden.

Komponente (a) wird in der erfindungsgemäßen Farbträgermasse in einer Gesamtmenge von 6,1 bis 25 Gewichtsprozent, vorzugsweise in einer Gesamtmenge von 7 bis 20 Gewichtsprozent, besonders bevorzugt in einer Gesamtmenge von 8 bis 15 Gewichtsprozent eingesetzt.

Komponente (b) wird in der erfindungsgemäßen Farbträgermasse in einer Gesamtmenge von 1 bis 20 Gewichtsprozent, vorzugsweise in einer Gesamtmenge von 2 bis 15 Gewichtsprozent, besonders bevorzugt in einer Gesamtmenge von 4 bis 10 Gewichtsprozent eingesetzt.

Komponente (c) wird in der erfindungsgemäßen Farbträgermasse in einer Gesamtmenge von 1 bis 15 Gewichtsprozent, vorzugsweise in einer Gesamtmenge von 2 bis 10 Gewichtsprozent, besonders bevorzugt in einer Gesamtmenge von 3 bis 6 Gewichtsprozent eingesetzt.

Die erfindungsgemäße Farbträgermasse enthält vorzugsweise Oxidationsfarbstoffvorstufen, bei denen die Färbung unter Einwirkung von Oxidationsmitteln, wie zum Beispiel Wasserstoffperoxid und dessen Addukten, oder in Gegenwart von Luftsauerstoff erzeugt wird.

Als geeignete Oxidationsfarbstoffvorstufen können beispielsweise die folgenden Entwicklersubstanzen und Kupplersubstanzen und mit sich selbst kuppelnden Verbindungen genannt werden:
(i) Entwicklersubstanzen: 1,4-Diamino-benzol (p-Phenylendiamin), 1,4-Diamino-2-methyl-benzol (p-Toluylendiamin), 1,4-Diamino-2,6-dimethyl-benzol, 1,4-Diamino-3,5-diethyl-benzol, 1,4-Diamino-2,5-dimethyl-benzol, 1,4-Diamino-2,3-dimethyl-benzol, 2-Chlor-1,4-diamino-benzol, 1,4-Diamino-2-(thiophen-2-yl)benzol, 1,4-Diamino-2-(thiophen-3-yl)benzol, 1,4-Diamino-2-(pyridin-3-yl)benzol, 2,5-Diamino-biphenyl, 1,4-Diamino-2-methoxymethyl-benzol, 1,4-Diamino-2-aminomethylbenzol, 1,4-Diamino-2-hydroxymethyl-benzol, 1,4-Diamino-2-(2-hydroxyethoxy)-benzol, 2-(2-(Acetylamino)ethoxy)-1,4-diamino-benzol, 4-Phenylamino-anilin, 4-Dimethylamino-anilin, 4-Diethylamino-anilin, 4-Dipropylamino-anilin, 4-[Ethyl(2-hydroxyethyl)amino]-anilin, 4-[Di(2-hydroxyethyl)amino]-anilin, 4-[Di(2-hydroxyethyl)amino]-2-methylanilin, 4-[(2-Methoxyethyl)amino]-anilin, 4-[(3-Hydroxypropyl)amino]-anilin, 4-[(2,3-Dihydroxypropyl)amino]-anilin, 1,4-Diamino-2-(2-hydroxyethyl)-benzol, 1,4-Diamino-2-(1-methylethyl)-benzol, 1,3-Bis[(4-aminophenyl)(2-hydroxyethyl)amino]-2-propanol, 1,4-Bis[(4-Aminophenyl)amino]-butan, 1,8-Bis(2,5-diaminophenoxy)-3,6-dioxaoctan, 4-Amino-phenol, 4-Amino-3-methyl-phenol, 4-Amino-3-(hydroxymethyl)-phenol, 4-Amino-3-fluorphenol, 4-Methylamino-phenol, 4-Amino-2-(aminomethyl)-phenol, 4-Amino-2-(hydroxymethyl)-phenol, 4-Amino-2-fluor-phenol, 4-Amino-2-[(2-hydroxyethyl)-amino]methyl-phenol, 4-Amino-2-methyl-phenol, 4-Amino-2-(methoxymethyl)-phenol, 4-Amino-2-(2-hydroxyethyl)-phenol, 5-Amino-salicylsäure, 2,5-Diamino-pyridin, 2,4,5,6-Tetraamino-pyrimidin, 2,5,6-Triamino-4-(1 H)-pyrimidon, 4,5-Diamino-1-(2-hydroxyethyl)-1 H-pyrazol, 4,5-Diamino-1-(1-methylethyl)-1 H-pyrazol, 4,5-Diamino-1-[(4-methylphenyl)methyl]-1 H-pyrazol, 1-[(4-Chlorphenyl)methyl]-4,5-diamino-1 H-pyrazol, 4,5-Diamino-1-methyl-1 H-pyrazol, 2-Amino-phenol, 2-Amino-6-methyl-phenol, 2-Amino-5-methyl-phenol, allein oder im Gemisch miteinander.
(ii) Kupplersubstanzen: N-(3-Dimethylamino-phenyl)-harnstoff, 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methylbenzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxybenzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 1,3-Diamino-4-(2,3-dihydroxypropoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)-amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methyl-phenol, 5-Amino-4-methoxy-2-methyl-phenol, 5-Amino-4-ethoxy-2-methyl-phenol, 3-Amino-2,4-dichlor-phenol, 5-Amino-2,4-dichlor-phenol, 3-Amino-2-methyl-phenol, 3-Amino-2-chlor-6-methyl-phenol, 3-Aminophenol, 2-[(3-Hydroxyphenyl)amino]-acetamid, 5-[(2-Hydroxyethyl)amino]-4-methoxy-2-methyl-phenol, 5-[(2-Hydroxyethyl)amino]-2-methyl-phenol, 3-[(2-Hydroxyethyl)amino]-phenol, 3-[(2-Methoxyethyl)amino]-phenol, 5-Amino-2-ethyl-phenol, 5-Amino-2-methoxy-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)-amino]-2-methyl-phenol, 2-Amino-3-hydroxy-pyridin, 5-Amino-4-chlor-2-methyl-phenol, 1-Naphthol, 2-Methyl-1-naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphthol-acetat, 1,3-Dihydroxybenzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxybenzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diamino-benzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol, 2,3-Indolindion, allein oder im Gemisch miteinander.
(iii) Mit sich selbst kuppelnde Verbindungen: 2-Amino-5-methylphenol, 2-Amino-6-methylphenol, 2-Amino-5-ethoxyphenol oder 2-Propylamino-5-aminopyridin.

Die Gesamtmenge der in der erfindungsgemäßen Farbträgermasse enthaltenen Oxidationsfarbstoffvorstufen beträgt etwa 0,01 bis 12 Gewichtsprozent, insbesondere etwa 0,2 bis 8 Gewichtsprozent.

Zur Erzielung bestimmter Farbnuancen können ferner auch übliche natürliche und/oder synthetische direktziehende Farbstoffe, beispielsweise sogenannte Pflanzenfarbstoffe wie Henna oder Indigo, Triphenylmethanfarbstoffe, aromatische Nitrofarbstoffe, Azofarbstofte, Chinonfarbstoffe, kationische oder anionische Farbstoffe, in dem Färbemittel enthalten sein.

Als geeignete synthetische Farbstoffe können beispielsweise genannt werden: 1,4-Bis[(2-hydroxyethyl)amino]-2-nitrobenzol, 1-(2-Hydroxyethyl)-amino-2-nitro-4-[di(2-hydroxyethyl)-amino]-benzol (HC Blue No. 2), 1-Amino-3-methyl-4-[(2-hydroxyethyl)-amino]-6-nitrobenzol (HC Violet No. 1), 4-[Ethyl-(2-hydroxyethyl)amino]-1-[(2-hydroxyethyl)amino]-2-nitrobenzol-hydrochlorid (HC Blue No. 12), 4-[Di(2-hydroxyethyl)amino]-1-[(2-methoxyethyl)amino]-2-nitrobenzol (HC Blue No. 11), 1-[(2,3-Dihydroxypropyl)amino]-4-[methyl-(2-hydroxy-ethyl)-amino]-2-nitrobenzol (HC Blue No. 10), 1-[(2,3-Dihydroxypropyl)-amino]-4-[ethyl-(2-hydroxyethyl)amino]-2-nitrobenzol-Hydrochlorid (HC Blue No. 9), 1-(3-Hydroxypropylamino)-4-[di(2-hydroxyethyl)amino]-2-nitrobenzol (HC Violet No. 2), 1-Methylamino-4-[methyl-(2,3-dihydroxy-propyl)amino]-2-nitrobenzol (HC Blue No. 6), 2-((4-Amino-2-nitrophenyl)-amino)-5-dimethylamino-benzoesäure (HC Blue No. 13), 1-Amino-4-[(2-hydroxyethyl)amino]-2-nitrobenzol (HC Red No. 7), 2-Amino-4,6-dinitro-phenol, 4-Amino-2-nitrodiphenylamin (HC Red No. 1), 1-Amino-4-[di(2-hydroxyethyl)amino]-2-nitrobenzol-Hydrochlorid (HC Red No. 13), 1-Amino-5-chlor-4-[(2-hydroxyethyl)amino]-2-nitrobenzol, 4-Amino-1-[(2-hydroxyethyl)amino]-2-nitrobenzol (HC Red No. 3),'4-Amino-3-nitrophenol, 4-[(2-Hydroxyethyl)-amino]-3-nitrophenol, 1-[(2-Aminoethyl)amino]-4-(2-hydroxyethoxy)-2-nitrobenzol (HC Orange No. 2), 4-(2,3-Dihydroxy-propoxy)-1-[(2-hydroxyethyl)amino]-2-nitrobenzol (HC Orange No. 3), 1-Amino-5-chlor-4-[(2,3-dihydroxypropyl)amino]-2-nitrobenzol (HC Red No. 10), 5-Chlor-1,4-[di(2,3-dihydroxypropyl)amino]-2-nitrobenzol (HC Red No. 11), 2-[(2-Hydroxyethyl)amino]-4,6-dinitro-phenol, 4-Ethylamino-3-nitrobenzoesäure, 2-[(4-Amino-2-nitrophenyl)-amino]-benzoesäure, 2-Chlor-6-ethylamino-4-nitrophenol, 2-Amino-6-chlor-4-nitrophenol, 4-[(3-Hydroxypropyl)amino]-3-nitrophenol, 2,5-Diamino-6-nitropyridin, 1,2,3,4-Tetrahydro-6-nitrochinoxalin, 7-Amino-3,4-dihydro-6-nitro-2H-1,4-benzoxazin (HC Red No. 14), 1-Amino-2-[(2-hydroxyethyl)amino]-5-nitrobenzol (HC Yellow No. 5), 1-(2-Hydroxy-ethoxy)-2-[(2-hydroxyethyl)-amino]-5-nitrobenzol (HC Yellow No. 4), 1-[(2-Hydroxyethyl)amino]-2-nitrobenzol (HC Yellow No. 2), 2-[(2-Hydroxy-ethyl)-amino]-1-methoxy-5-nitrobenzol, 2-Amino-3-nitrophenol, 1-(2-Hydroxyethoxy)-3-methylamino-4-nitrobenzol, 2,3-(Dihydroxy-propoxy)-3-methyl-amino-4-nitrobenzol, 2-[(2-Hydroxyethyl)amino]-5-nitrophenol (HC Yellow No. 11), 3-[(2-Aminoethyl)-amino]-1-methoxy-4-nitrobenzol-Hydrochlorid (HC Yellow No.9), 1-[(2-Ureidoethyl)amino]-4-nitrobenzol, 4-[(2,3-Di-hydroxypropyl)amino]-3-nitro-1-trifluormethyl-benzol (HC Yellow No. 6), 1-Chlor-2,4-bis[(2-hydroxyethyl)-amino]-5-nitrobenzol (HC Yellow No. 10), 4-[(2-Hydroxy-ethyl)amino]-3-nitro-1-methylbenzol, 1-Chlor-4-[(2-hydroxy-ethyl)amino]-3-nitrobenzol (HC Yellow No. 12), 4-[(2-Hydroxyethyl)-amino]-3-nitro-1-trifluormethyl-benzol (HC Yellow No. 13), 4-[(2-Hydroxy-ethyl)amino]-3-nitro-benzonitril (HC Yellow No. 14), 4-[(2-Hydroxyethyl)-amino]-3-nitro-benzamid (HC Yellow No. 15), 1,4-Di[(2,3-dihydroxy-propyl)amino]-9,10-anthrachinon, 1-[(2-Hydroxy-ethyl)amino]-4-methyl-amino-9,10-anthrachinon (Cl61505, Disperse Blue No. 3), 2-[(2-Amino-ethyl)amino]-9,10-anthrachinon (HC Orange No. 5), 1-Hydroxy-4-[(4-methyl-2-sulfophenyl)amino]-9,10-anthrachinon, 1-[(3-Aminopropyl)-amino]-4-methylamino-9,10-anthrachinon (HC Blue No. 8),1-[(3-Amino-propyl)-amino]-9,10-anthrachinon (HC Red No. 8), 1,4-Diamino-2-methoxy-9,10-anthrachinon (Cl62015, Disperse Red No. 11, Solvent Violet No. 26), 1,4-Dihydroxy-5,8-bis[(2-hydroxyethyl)amino]-9,10-anthrachinon (Cl62500, Disperse Blue No. 7, Solvent Blue No. 69), 9-(Dimethylamino)-benzo[a]-phenoxazin-7-ium-chlorid (Cl51175; Basic Blue No. 6), Di[4-(diethyl-amino)phenyl][4-(ethylamino)naphthyl]-carbeniumchlorid (Cl42595; Basic Blue No. 7), 3,7-Di(dimethylamino)-phenothiazin-5-ium-chlorid (Cl52015; Basic Blue No. 9), Di[4-(dimethyl-amino)phenyl][4-(phenylamino)naphthyl]-carbenium-chlorid (Cl44045; Basic Blue No. 26), 2-[(4-(Ethyl(2-hydroxy-ethyl)amino)-phenyl)azo]-6-methoxy-3-methyl-benzothiazolium-methylsulfat (Cl11154; Basic Blue No. 41), 8-Amino-2-brom-5-hydroxy-4-imino-6-[(3-(trimethylammonio)phenyl)-amino]-1(4H)-naphthalinon-chlorid (Cl56059; Basic Blue No. 99), Bis[4-(dimethyl-amino)phenyl][4-(methyl-amino)phenyl]-carbenium-chlorid (Cl42535; Basic Violet No. 1), Tris[4-(dimethylamino)-phenyl]carbenium-chlorid (Cl42555; Basic Violet No. 3), 2-[3,6-(Diethylamino)-dibenzopyranium-9-yl]-benzoesäure-chlorid (Cl45170; Basic Violet No. 10), Di(4-aminophenyl)-(4-amino-3-methyl-phenyl)carbenium-chlorid (Cl42510; Basic Violet No. 14), 1,3-Bis[(2,4-diamino-5-methylphenyl)azo]-3-methylbenzol (Cl21010; Basic Brown No. 4), 1-[(4-Aminophenyl)azo]-7-(trimethylammonio)-2-naphthol-chlorid (Cl12250; Basic Brown No. 16), 1-[(4-Amino-2-nitrophenyl)azo]-7-(trimethylammonio)-2-naphthol-chlorid (Basic Brown No. 17), 1-[(4-Amino-3-nitrophenyl)azo]-7-(trimethyl-ammonio)-2-naphthol-chlorid (Cl12251; Basic Brown No. 17), 3,7-Diamino-2,8-dimethyl-5-phenyl-phenazinium-chlorid (Cl50240; Basic Red No. 2), 1,4-Dimethyl-5-[(4-(dimethylamino)phenyl)-azo]-1,2,4-triazolium-chlorid (Cl11055; Basic Red No. 22), 2-Hydroxy-1-[(2-methoxyphenyl)azo]-7-(trimethylammonio)-naphthalin-chlorid (Cl12245; Basic Red No. 76), 2-[2-((2,4-Dimethoxy-phenyl)amino)ethenyl]-1,3,3-trimethyl-3H-indol-1-ium-chlorid (Cl48055; Basic Yellow No. 11), 3-Methyl-1-phenyl-4-[(3-(trimethylammonio)-phenyl)azo]-pyrazol-5-on-chlorid (Cl12719; Basic Yellow No. 57), Bis[4-(diethylamino)phenyl]phenylcarbenium-Hydrogensulfat(1:1) (Cl42040; Basic Green No. 1), 1-[Di(2-hydroxyethyl)-amino]-3-methyl-4-[(4-nitro-phenyl)azo]-benzol (Cl11210, Disperse Red No. 17), 4-[(4-Aminophenyl)-azo]-1-[di(2-hydroxyethyl)amino]-3-methylbenzol (HC Yellow No. 7), 2,6-Diamino-3-[(pyridin-3-yl)azo]-pyridin, 6-Hydroxy-5-[(4-sulfophenyl)azo]-2-naphthalinsulfonsäure-Dinatriumsalz (Cl15985; Food Yellow No. 3; FD&C Yellow No. 6), 2,4-Dinitro-1-naphthol-7-sulfonsäure-Dinatriumsalz (Cl10316; Acid Yellow No. 1; Food Yellow No. 1), 2-(Indan-1,3-dion-2-yl)chinolin-x,x-sulfonsäure (Gemisch aus Mono- und Disulfonsäure) (Cl47005;D&C Yellow No. 10; Food Yellow No. 13; Acid Yellow No. 3), 5-Hydroxy-1-(4-sulfophenyl)-4-[(4-sulfophenyl)-azo]pyrazol-3-carbonsäure-Trinatriumsalz (Cl19140; Food Yellow No. 4; Acid Yellow No. 23), 9-(2-Carboxyphenyl)-6-hydroxy-3H-xanthen-3-on (Cl45350; Acid Yellow No. 73; D&C Yellow No. 8), 5-[(2,4-Dinitrophenyl)-amino]-2-phenylamino-benzolsulfonsäure-Natriumsalz (Cl10385; Acid Orange No. 3), 4-[(2,4-Dihydroxyphenyl)azo]-benzolsulfonsäure-Mononatriumsalz (Cl14270; Acid Orange No. 6), 4-[(2-Hydroxynaphth-1-yl)azo]-benzolsulfonsäure-Natriumsalz (Cl15510; Acid Orange No. 7), 4-[(2,4-Dihydroxy-3-[(2,4-dimethylphenyl)azo]phenyl)azo]-benzolsulfonsäure-Natriumsalz (Cl20170; Acid Orange No. 24), 4-Hydroxy-3-[(4-sulfonaphth-1-yl)azo]-1-naphthalin-sulfonsäure-Dinatriumsalz (Cl14720; Acid Red No. 14), 6-Hydroxy-5-[(4-sulfonaphth-1-yl)azo]-2, 4-naphthalindisulfonsäure-Trinatriumsalz (Cl16255; Ponceau 4R; Acid Red No. 18), 3-Hydroxy-4-[(4-sulfonaphth-1-yl)azo]-2,7-naphthalin-disulfonsäure-Trinatriumsalz (Cl16185; Acid Red No. 27), 8-Amino-1-hydroxy-2-(phenylazo)-3,6-naphthalin-disulfonsäure-Dinatriumsalz (Cl17200; Acid Red No. 33), 5-(Acetylamino)-4-hydroxy-3-[(2-methylphenyl)azo]-2,7-naphthalin-disulfonsäure-Dinatriumsalz (Cl18065; Acid Red No. 35), 2-(3-Hydroxy-2,4,5,7-tetraiod-dibenzopyran-6-on-9-yl)-benzoesäure-Dinatriumsalz (Cl45430;Acid Red No. 51), N-[6-(Diethylamino)-9-(2,4-disulfophenyl)-3H-xanthen-3-yliden]-N-ethylethanammoniumhydroxid, inneres Salz, Natriumsalz (Cl45100; Acid Red No. 52), 8-[(4-(Phenylazo)-phenyl)azo]-7-naphthol-1,3-disulfonsäure-Dinatriumsalz (Cl27290; Acid Red No. 73), 2',4',5',7'-Tetrabrom-3',6'-dihydroxyspiro-[isobenzofuran-1(3H),9'-[9H]xanthen]-3-on-Dinatriumsalz (Cl45380; Acid Red No. 87), 2',4',5',7'-Tetrabrom-4,5,6,7-tetrachlor-3',6'-dihydroxyspiro[isobenzofuran-1(3H),9'[9H]xanthen]-3-on-Dinatriumsalz (Cl45410; Acid Red No. 92), 3',6'-Dihydroxy-4',5'-diiodospiro[isobenzofuran-1 (3H),9'(9H)-xanthen]-3-on-Dinatriumsalz (Cl45425; Acid Red No. 95), (2-Sulfophenyl)di[4-(ethyl((4-sulfophenyl)methyl)amino)phenyl]-carbenium-Dinatriumsalz, Betain (Cl42090; Acid Blue No. 9; FD&C Blue No. 1), 1,4-Bis[(2-sulfo-4-methylphenyl)amino]-9,10-anthrachinon-Dinatriumsalz (Cl61570; Acid Green No. 25), Bis[4-(dimethylamino)phenyl]-(3,7-disulfo-2-hydroxy-naphth-1-yl)carbenium-inneres Salz, Mononatriumsalz (Cl44090; Food Green No. 4; Acid Green No. 50), Bis[4-(diethylamino)phenyl](2,4-disulfophenyl)-carbenium-inneres Salz, Natriumsalz (2:1) (Cl42045; Food Blue No. 3; Acid Blue No. 1), Bis[4-(diethylamino)phenyl](5-hydroxy-2,4-disulfophenyl)carbenium-inneres Salz, Calciumsalz (2:1) (Cl42051; Acid Blue No. 3), 1-Amino-4-(cyclohexylamino)-9,10-anthrachinon-2-sulfonsäure-Natriumsalz (Cl62045; Acid Blue No. 62),2-(1,3-Dihydro-3-oxo-5-sulfo-2H-indol-2-yliden)-2,3-dihydro-3-oxo-1 H-indol-5-sulfonsäure-Dinatriumsalz (Cl73015; Acid Blue No. 74), 9-(2-Carboxyphenyl)-3-[(2-methylphenyl)amino]-6-[(2-methyl-4-sulfophenyl)amino]xanthylium-inneres Salz, Mononatriumsalz (Cl45190; Acid Violet No. 9), 1-Hydroxy-4-[(4-methyl-2-sulfophenyl)amino]-9,10-anthrachinon-Natriumsalz (Cl60730; D&C Violett No. 2; Acid Violet No. 43), Bis[3-nitro-4-[(4-phenylamino)-3-sulfo-phenylamino]-phenyl]-sulfon (Cl10410; Acid Brown No. 13), 5-Amino-4-hydroxy-6-[(4-nitrophenyl)azo]-3-(phenylazo)-2,7-naphthalindisulfonsäure-Dinatriumsalz (Cl20470; Acid Black No. 1), 3-Hydroxy-4-[(2-hydroxynaphth-1-yl)azo]-7-nitro-1-naphthalin-sulfonsäure-Chromkomplex (3:2) (Cl15711; Acid Black No. 52), 3-[(2,4-Dimethyl-5-sulfophenyl)azo]-4-hydroxy-1-naphthalin-sulfonsäure-Dinatriumsalz (Cl14700; Food Red No. 1; FD&C Red No. 4), 4-(Acetylamino)-5-hydroxy-6-[(7-sulfo-4-[(4-sulfophenyl)azo]naphth-1-yl)azo]-1,7-naphthalindisulfonsäure-Tetranatriumsalz (Cl28440; Food Black No. 1) und 3-Hyd roxy-4-(3-methyl-5-oxo-1-phenyl-4,5-dihydro-1 H-pyrazol-4-ylazo)-naphthalin-1-sulfonsäure-Natriumsalz, Chrom-Komplex (Acid Red No. 195), alleine oder in Kombination miteinander.

Die Gesamtmenge der direktziehenden Farbstoffe beträgt in der erfindungsgemäßen Farbträgermasse etwa 0,01 bis 7 Gewichtsprozent, vorzugsweise etwa 0,2 bis 4 Gewichtsprozent.

Weitere zur Haarfärbung bekannte und übliche Farbstoffe, die in dem erfindungsgemäßen Färbemittel enthalten sein können, sind unter anderem in E. Sagarin, "Cosmetics, Science and Technology", Interscience Publishers Inc., New York (1957), Seiten 503 ff. sowie H. Janistyn, "Handbuch der Kosmetika und Riechstoffe", Band 3 (1973), Seiten 388 ff. und K. Schrader "Grundlagen und Rezepturen der Kosmetika", 2. Auflage (1989), Seiten 782 bis 815 beschrieben, auf die hiermit ausdrücklich Bezug genommen wird.

Obwohl die erfindungsgemäße Farbträgermasse besonders bevorzugt für Oxidationsfärbemittel geeignet ist, können selbstverständlich ebenfalls nicht-oxidative Färbemittel auf Basis der vorstehend genannten direktziehenden Farbstoffe mit der erfindungsgemäßen Farbträgermasse erstellt werden.

Zur weiteren Verbesserung der Ausspülbarkeit nach der Anwendung kann das erfindungsgemäße Färbemittel neben den Verbindungen der Komponente (c) zusätzlich weitere Tenside enthalten. Vorzugsweise sind nichtionische und amphotere Tenside sowie deren Kombinationen im erfindungsgemäßen Färbemittel enthalten, weil diese eine ausgezeichnete Verträglichkeit gegenüber weiteren ionogenen - sowohl anionischen wie kationischen -Additiven ermöglichen. Außerdem kann durch die Verwendung von nichtionischen und amphoteren Tensiden sowie deren Kombinationen der Pflegeeffekt weiter verstärkt werden.

Erfindungsgemäß geeignete amphotere Tenside sind Acylethyldiamine und deren Derivate, N-Alkylaminosäuren oder Imino-Dicarbonsäuren sowie insbesondere Betaine wie zum Beispiel Cocamidopropylbetain. Erfindungsgemäß geeignete nichtionische Tenside sind Polyethylenglykole, Polypropylenglykole, Ethylenoxid/Propylenoxid-Blockpolymere, Alkylphenolethoxylate, Alkylpolyglucoside, ethoxylierte Alkanolamide, Glykol- und Glycerolester sowie deren Alkoxlate, Sorbitanester und deren Alkoxylate, Alkylcarbohydratester (Zuckerester), Alkylcarbohydratether (Zuckerether), ethoxylierte Pentaerythritester sowie alkoxylierte Polysiloxane.

Eine ausführliche Beschreibung dieser nichtionischen oder amphoteren Tenside ist der Publikation "FIEDLER - Lexikon der Hilfsstoffe", Band 1, fünfte Auflage (2002), Seiten 105 bis 113, zu entnehmen, auf die hiermit ausdrücklich Bezug genommen wird.

Das erfindungsgemäße Färbemittel kann diese zusätzlichen Tenside in einer Gesamtmenge von 0,1 bis 10 Gewichtsprozent, bevorzugt jedoch 0,5 bis 6 Gewichtsprozent enthalten.

In der erfindungsgemäßen Farbträgermasse können darüberhinaus Antioxidantien wie zum Beispiel Ascorbinsäure, Thioglykolsäure oder Natriumsulfit, sowie Komplexbildner für Schwermetalle, beispielsweise Ethylendiaminotetraacetat oder Nitriloessigsäure, in einer Menge von bis zu etwa 1,5 Gewichtsprozent enthalten sein. Parfümöle können in der erfindungsgemäßen Farbträgermasse in einer Menge von bis zu etwa 1,5 Gewichtsprozent enthalten sein. Selbstverständlich kann die vorstehend beschriebene Farbträgermasse gegebenenfalls weitere, für Haarfärbemittel übliche Zusätze, wie zum Beispiel Verdickungsmittel, beispielsweise Homopolymere der Acrylsäure, Pflanzen Gums, Cellulose- und Stärkederivate, Algenpolyasaccharide, amphiphile Assoziatiwerdicker, desweiteren Konservierungsstoffe Antioxidantien, beispielsweise Natriumsulfit, Thioglykolsäure und Ascorbinsäure oder deren Mischungen, Komplexbildner, Lösungsmittel wie Wasser, niedere aliphatische Alkohole, beispielsweise aliphatische Alkohole mit 1 bis 4 Kohlenstoffatomen wie Ethanol, Propanol und Isopropanol, oder Glykole wie Glycerin und 1,2-Propylenglykol, weitere Netzmittel oder Emulgatoren, weiterhin Weichmacher, Vaseline, Silikonöle, Paraffinöl, Polysorbate und Fettsäuren sowie außerdem Pflegestoffe, wie kationische Polymere oder Harze, Lanolinderivate, Cholesterin, Vitamine, Pantothensäure oder Betain, enthalten. Die erwähnten Bestandteile werden in den für solche Zwecke üblichen Mengen verwendet, zum Beispiel die Netzmittel und Emulgatoren in Konzentrationen von 0,1 bis 30 Gewichtsprozent und die Pflegestoffe in einer Konzentration von 0,1 bis 5,0 Gewichtsprozent.

Vorzugsweise ist das erfindungsgemäße Färbemittel frei von Glycoldistearat.

Der pH-Wert der erfindungsgemäßen Farbträgermasse liegt bei nichtoxidativen Färbemitteln auf der Basis von direktziehenden Farbstoffen im Bereich von etwa 5 bis 10, vorzugsweise 6 bis 9, während bei oxidativen Färbemitteln auf der Basis von Oxidationsfarbstoffvorstufen der pH-Wert in einem Bereich von etwa 6 bis 12, vorzugsweise 9 bis 11, liegt, wobei der pH-Wert des gebrauchsfertigen Oxidationshaarfärbemittels (das heißt der Mischung der erfindungsgemäßen Farbträgermasse mit dem Oxidationsmittel) etwa 5,5 bis 10,5, vorzugsweise 6 bis 10, beträgt.

Je nach Zusammensetzung und gewünschtem pH-Wert erfolgt die Einstellung des pH-Wertes vorzugsweise mit Ammoniak, Aminosäuren oder organischen Aminen, wie zum Beispiel Glucaminen, Aminomethylpropanol, Monoethanolamin oder Triethanolamin, anorganischen Basen, beispielsweise Natriumhydroxid, Kaliumhydroxid, Natriumcarbonat, Natrium-metasilikat oder Calciumhydroxid, beziehungsweise organischen oder anorganischen Säuren, wie zum Beispiel Milchsäure, Zitronensäure, Essigsäure oder Phosphorsäure.

Die erfindungsgemäße Farbträgermasse wird vorzugsweise in Form einer wässrigen oder wässrig-alkoholischen Zubereitung, beispielsweise als verdickte Lösung, als Emulsion, als Creme oder als Gel, konfektioniert.

Für die Anwendung zur oxidativen Färbung vermischt man die vorstehend beschriebene Farbträgermasse unmittelbar vor dem Gebrauch mit einem Oxidationsmittel und trägt eine für die Färbung ausreichende Menge, in der Regel etwa 60 bis 200 Gramm, der gebrauchsfertigen Zubereitung auf die Faser auf.

Sofern die erfindungsgemäße Farbträgermasse keine Oxidationsfarbstoffvorstufen enthält beziehungsweise Oxidationsfarbstoffvorstufen enthält, welche mit Luftsauerstoff leicht oxidierbar sind, kann sie ohne vorheriges Vermischen mit einem Oxidationsmittel direkt auf die Keratinfaser aufgetragen werden.

Als Oxidationsmittel zur Entwicklung der Färbung kommen hauptsächlich Wasserstoffperoxid oder dessen Additionsverbindungen an Harnstoff, Melamin oder Natriumborat in Form einer 1- bis 12prozentigen, vorzugsweise 1,5- bis 6prozentigen wässrigen Lösung in Betracht. Das Mischungsverhältnis von Farbträgermasse zu Oxidationsmittel ist abhängig von der Konzentration des Oxidationsmittels und beträgt in der Regel etwa 5:1 bis 1:3, vorzugsweise 1:1, wobei der Gehalt an Oxidationsmittel in der gebrauchsfertigen Zubereitung vorzugsweise etwa 0,5 bis 8 Gewichtsprozent, insbesondere 1 bis 4 Gewichtsprozent, beträgt.

Man läßt das gebrauchsfertige Färbemittel bei 15 bis 50 °C etwa 10 bis 45 Minuten, vorzugsweise etwa 15 bis 30 Minuten lang, auf die Keratinfaser (zum Beispiel menschliche Haare) einwirken, spült sodann die Faser mit Wasser aus. Gegebenenfalls wird im Anschluß an diese Spülung mit einem Shampoo gewaschen und eventuell mit einer schwachen organischen Säure, wie zum Beispiel Weinsäure, nachgespült. Abschließend wird die Keratinfaser getrocknet.

Die erfindungsgemäße Farbträgermasse weist eine gleichmäßige Konsistenz sowie eine sehr kosmetische perlmuttglänzende Anmutung auf. Ein mit der erfindungsgemäßen Farbträgermasse hergestelltes Färbemittel erfüllt die in Bezug auf die Hafteigenschaften, das Auftrageverhalten und die Viskositätseinstellung gestellten Anforderungen in hervorragenderer Weise und bietet durch den perlmuttartigen Charakter ein überaus kosmetisches Aussehen.

Ein weiterer Gegenstand der vorliegenden Anmeldung ist die Verwendung einer Kombination aus
(a) 6,1 bis 25 Gewichtsprozent, insbesondere 7 bis 20 Gewichtsprozent, besonders bevorzugt 8 bis 15 Gewichtsprozent, mindestens eines Fettalkoholes mit 14 bis 20 Kohlenstoffatomen,
(b) 1 bis 20 Gewichtsprozent, insbesondere 2 bis 15 Gewichtsprozent, besonders bevorzugt 4 bis 10 Gewichtsprozent, mindestens eines Fettsäurealkanolamides und
(c) 1 bis 15 Gewichtsprozent, insbesondere 2 bis 10 Gewichtsprozent, besonders bevorzugt 3 bis 6 Gewichtsprozent, mindestens eines Fettalkoholalkoxylates und/oder Fettsäurealkoxylates,
wobei das Gewichtsverhältnis von Fettalkohol (a) zu Alkanolamid (b) in der Farbträgermasse gleich 3:1 bis 1:2, insbesondere gleich 2,5:1 bis 1:1 ist und gleichzeitig das Verhältnis von Fettalkohol (a) zu Alkoxylat (c) gleich 4:1 bis 1:1,5, insbesondere gleich 3:1 bis 1:1 ist und keine monomeren quaternären Ammoniumverbindungen sowie anionischen und kationischen Emulgatoren und Tensiden enthalten sind,
zur Erzeugung eines Perlglanzeffektes in Farbträgermassen und Färbemitteln für Keratinfasern, insbesondere menschlichen Haaren.

Zur weiteren Verbesserung der Ausspülbarkeit nach der Anwendung können der vorstehenden Kombination neben den Verbindungen der Komponente (c) zusätzlich weitere Tenside zugesetzt werden. Vorzugsweise werden nichtionische und amphotere Tenside sowie deren Kombinationen zugesetzt, weil diese eine ausgezeichnete Verträglichkeit gegenüber weiteren ionogenen - sowohl anionischen wie kationischen - Additiven ermöglichen. Außerdem kann durch die Verwendung von nichtionischen und amphoteren Tensiden sowie deren Kombinationen der Pflegeeffekt weiter verstärkt werden.

Die nachfolgenden Beispiele sollen den Gegenstand der Erfindung näher erläutern ohne diesen hierauf zu beschränken.

### Beispiele

| **Beispiel 1:** | **Oxidationshaarfärbemittel, cremeförmig** |
|---|---|
| 8,00 g | Stearylalkohol |
| 4,00 g | Cetearylalkohol |
| 1,00 g | Propylenglycol |
| 6,00 g | Kokosfettsäuremonoethanolamid (Cocamide MEA) |
| 2,50 g | Ceteareth-25 |
| 2,00 g | Steareth-20 |
| 4,00 g | Cocamidopropyl Betain (30%-ige wässrige Lösung) |
| 1,90 g | 2,5-Diamino-toluol-sulfat |
| 0,40 g | Resorcin |
| 0,10 g | m-Aminophenol |
| 0,12 g | 2-Methyl-resorcin |
| 0,10 g | a-Naphthol |
| 0,10 g | 2-Amino-4-hydroxyethylaminoanisol Sulfat |
| 0,10 g | HC Yellow No.13 |
| 0,60 g | 2,4-Diaminophenoxyethanol Sulfat |
| 4,55 g | Ammoniak, 25%ige wässrige Lösung |
| 0,30 g | Ethylendiaminoteraacetat-Dinatriumsalz |
| 0,40 g | Ascorbinsäure |
| 0,50 g | Polyquarternium-11 |
| 2,00 g | Alanin |
| ad 100,00 g | Wasser |

50 g der vorstehenden perlmuttglänzenden Farbträgermasse werden unmittelbar vor Gebrauch mit 50 g einer 6prozentigen wässrigen Wasserstoffperoxidlösung vermischt. Es wird eine homogene, perlmuttgänzende, kosmetisch anmutende Färbezubereitung erhalten. Das so erhaltene Gemisch wird anschließend auf blonde Haare aufgetragen. Nach einer Einwirkungszeit von 30 Minuten bei 40 °C wird das Haar mit Wasser gespült und getrocknet. Das Haar erhält eine schwarze Färbung.

| **Beispiel 2:** | **Cremeförmiges Oxidationshaarfärbemittel** |
|---|---|
| Komponente (A): | Cremeförmige Farbträgermasse |
| 14,000 g | Cetearylalkohol |
| 2,500 g | PPG-10-Cetylether |
| 7,000 g | Kokosfettsäuremonoethanolamid (Cocamide MEA) |
| 3,000 g | Ceteareth-25 |
| 1,000 g | Ölsäure |
| 1,500 g | Polysorbat-60 |
| 0,260 g | 2,5-Diamino-toluol-sulfat |
| 0,140 g | Resorcin |
| 0,005 g | m-Aminophenol |
| 0,002 g | Amino-4-hydroxyethylaminoanisol-sulfat |
| 0,005 g | 2-Methyl-resorcin |
| 0,010 g | Hydroxyethyl-2-nitro-p-toluidine |
| 0,500 g | Ethylendiamintetraacetat-Dinatriumsalz |
| 16,000 g | Ammoniak, 25%ige wässrige Lösung |
| 3,000 g | Creatin |
| 3,000 g | Dimethyldiallylammoniumchlorid/Acrylamid-Copolymer (Polyquaternium-7) |
| ad 100,000 g | Wasser |

| Komponente (B): | Wasserstoffperoxid-Emulsion |
|---|---|
| 10,0 g | Cetylstearylalkohol |
| 1,5 g | Cholesterin |
| 4,0 g | Natriumlaurylalkoholdiglykolethersulfat, 28%ige wässrige Lösung |
| 35,0 g | Wasserstoffperoxid, 35%ige wässrige Lösung |
| 0,3 g | Parfüm |
| ad 100,0 g | Wasser |

Man vermischt vor dem Gebrauch 40 g der Farbträgermasse (A) mit 80 g der Wasserstoffperoxid-Emulsion (B), entsprechend einem Mischungsverhältnis von (A):(B) von 1:2, und trägt 120 g dieses Gemisches auf graues Haar auf. Nach einer Einwirkungszeit von 20 Minuten bei Raumtemperatur wird das Haar mit Wasser ausgespült und anschließend getrocknet. Das so behandelte Haar ist vom Haaransatz bis zu den Haarspitzen gleichmäßig hellblond gefärbt. Das erfindungsgemäße perlglänzende Mittel ist leicht auftragbar und läuft nicht vom Haar ab.

| **Beispiel 3:** | **Oxidationshaarfärbemittel, cremeförmig** |
|---|---|
| 14,00 g | Stearylakohol |
| 2,50 g | PEG-30-stearat |
| 10,00 g | Kokosfettsäuremonoethanolamid (Cocamide MEA) |
| 3,00 g | Ceteareth-25 |
| 5,00 g | Cocamidopropyl Betain (30%-ige wässrige Lösung) |
| 8,00 g | Monoethanolamin |
| 2,30 g | 1-Hydroxyethyl-4,5-diamino-pyrazol-sulfat |
| 1,19 g | 4-Amino-m-cresol |
| 0,10 g | HC Red No.10 |
| 0,20 g | 2-Amino-6-chloro-4-nitrophenol |
| 0,50 g | Keratinhydrolysat |
| 0,50 g | Seidenproteinhydrolysat |
| 0,50 g | Cyclomethicone |
| 0,50 g | Ethylendiaminotetraacetat-Dinatriumsalz |
| 0,30 g | Ascorbinsäure |
| 0,10 g | Natriumsulfit |
| ad 100,00 g | Wasser |

50 g der vorstehenden Farbträgermasse werden unmittelbar vor Gebrauch mit 50 g einer 12prozentigen wässrigen Wasserstoffperoxidlösung vermischt. Das erhaltene Gemisch wird anschließend auf mittelblonde Naturhaare aufgetragen. Nach einer Einwirkungszeit von 30 Minuten bei 40 °C wird das Haar mit Wasser gespült und getrocknet. Es wird ein gleichmäßiger, kräftiger orangeroter Farbton erhalten.

| **Beispiel 4:** | **Haarfärbemittel mit direktziehenden Farbstoffen** |
|---|---|
| 2,000 g | Cetearylalkohol |
| 8,000 g | Stearylalkohol |
| 2,000 g | Myristylalkohol |
| 6,000 g | Kokosfettsäuremonoethanolamid (Cocamide MEA) |
| 2,000 g | Steareth-20 |
| 3,000 g | Ceteth-20 |
| 5,000 g | Cocamidopropyl Hydroxysultaine |
| 2,000 g | Isopropylalkohol |
| 0,160 g | HC Blue 12 |
| 0,170 g | HC Yellow 13 |
| 0,012 g | Hydroxyethyl-2-nitro-toluidin |
| 0,035 g | HC RED NO. 10 und HC RED NO. 11 (1:1) |
| 1,000 g | Dimethyldiallylammoniumchlorid/Acrylamid-Copolymer (Polyquaternium-7) |
| 0,200 g | kationisches Cellulosederivat (Polyquaternium-10) |
| ad 100,000 g | Wasser |

Die perlglänzende cremeartige Färbemasse wird mit Handschuhen auf das gewaschene und handtuchtrockene blonde Naturhaar aufgetragen und 20 bis 25 Minuten einwirken gelassen. Die überschüssige Farbe wird mit Wasser und Shampoo herausgewaschen. Es wird ein schöner, glänzender mittelblonder Ton erhalten.

| **Beispiel 5:** | **Haarfärbemittel mit direktziehenden Farbstoffen** |
|---|---|
| 12,0 g | Stearylalkohol |
| 6,0 g | Kokosfettsäurediethanolamid (Cocamide DEA) |
| 2,5 g | PPG-5-Ceteth-20 |
| 2,5 g | Ceteareth-25 |
| 7,0 g | Ethanol, wässrig |
| 0,1 g | Hydroxyethyl-2-nitro-toluidin |
| 0,5 g | HC RED NO. 10 und HC RED NO. 11 (1:1) |
| 0,2 g | 2-Amino-6-chloro-4-nitrophenol |
| 2,0 g | Dimethyldiallylammoniumchlorid/Acrylamid-Copolymer (Polyquaternium-7) |
| 0,5 g | kationisches Cellulosederivat (Polyquaternium-10) |
| ad 100,0 g | Wasser |

Die perlglänzende cremeartige Färbemasse wird mit Handschuhen auf das gewaschene und handtuchtrockene blonde Naturhaar aufgetragen und 20 bis 25 Minuten einwirken gelassen. Die überschüssige Farbe wird mit Wasser und Shampoo herausgewaschen. Es wird ein schöner, glänzender rotblonder Ton erreicht.

Alle in der vorliegenden Anmeldung genannten Prozentangeaben stellen, soweit nicht anders angegeben, Gewichtsprozente dar.

## Patentansprüche

1. Farbträgermasse, enthaltend oxidative und/oder nicht-oxidative Farbstoffe, **dadurch gekennzeichnet, dass** sie frei von monomeren quaternären Ammoniumverbindungen sowie anionischen und kationischen Emulgatoren und Tensiden ist, und eine Kombination aus
(a) 6,1 bis 25 Gewichtsprozent mindestens eines Fettalkohols mit 14 bis 20 Kohlenstoffatomen,
(b) 1 bis 20 Gewichtsprozent mindestens eines Fettsäurealkanolamids und
(c) 1 bis 15 Gewichtsprozent mindestens eines Fettalkoholalkoxylats und/oder Fettsäurealkoxylats enthält,
wobei das Gewichtsverhältnis von Fettalkohol (a) zu Fettsäurealkanolamid (b) gleich 4:1 bis 1:3 und gleichzeitig das Gewichtsverhältnis von Fettalkohol (a) zu Alkoxylat (c) gleich 5:1 bis 1:2 ist.

2. Farbträgermasse nach Anspruch 1, **dadurch gekennzeichnet, dass** der Fettalkohol (a) und das Fettsäurealkanolamid (b) in einem Gewichtsverhältnis von 3:1 bis 1:2 vorliegen und gleichzeitig das Verhältnis von Fettalkohol (a) zu Alkoxylat (c) gleich 4:1 bis 1:1,5 ist.

3. Farbträgermasse nach Anspruch 1, **dadurch gekennzeichnet, dass** der Fettalkohol (a) und das Fettsäurealkanolamid (b) in einem Gewichtsverhältnis von 2,5:1 bis 1:1 vorliegen und gleichzeitig das Verhältnis von Fettalkohol (a) zu Alkoxylat (c) 3:1 bis 1:1 ist.

4. Farbträgermasse nach einem der Ansprüche 1 bis 3, **dadurch gekennzeichnet, dass** der Fettalkohol (a) ausgewählt ist aus Cetylakohol, Stearylalkohol, Myristylalkohol, Isooctylalkohol, Isotridecylalkohol sowie Mischungen dieser Verbindungen.

5. Farbträgermasse nach einem der Ansprüche 1 bis 4, **dadurch gekennzeichnet, dass** das Alkanolamid (b) ausgewählt ist aus N-Acylderivaten des Monoethanolamins oder Diethanolamins sowie den Estern des Monoethanolamins oder Diethanolamins.

6. Farbträgermasse nach einem der Ansprüche 1 bis 5, **dadurch gekennzeichnet, dass** das Alkoxylat (c) ausgewählt ist aus der Gruppe der Fettalkoholpolyalkylenglykolether und Fettsäurepolyalkylenglykolester mit 8 bis 30 Kohlenstoffatomen im Fettalkoholrest beziehungsweise Fettsäurerest und 2 bis 300 Alkylenglykoleinheiten im Polyalkylenglykolrest.

7. Farbträgermasse nach einem der Ansprüche 1 bis 6, **dadurch gekennzeichnet, dass** die Komponente (a) in einer Gesamtmenge von 6,1 bis 25 Gewichtsprozent enthalten ist.

8. Farbträgermasse nach einem der Ansprüche 1 bis 7, **dadurch gekennzeichnet, dass** die Komponente (b) in einer Gesamtmenge von 1 bis 20 Gewichtsprozent enthalten ist.

9. Farbträgermasse nach einem der Ansprüche 1 bis 8, **dadurch gekennzeichnet, dass** die Komponente (c) in einer Gesamtmenge von 1 bis 15 Gewichtsprozent enthalten ist.

10. Farbträgermasse nach einem der Ansprüche 1 bis 9, **dadurch gekennzeichnet, dass** sie neben den Verbindungen der Komponente (c) zusätzlich weitere nichtionische und/oder amphotere Tenside enthält.

11. Mittel zur oxidativen Färbung von Haaren, **dadurch gekennzeichnet, dass** es durch Vermischen einer Farbträgermasse nach einem der Ansprüche 1 bis 10 mit einem Oxidationsmittel erhalten wird.

12. Mittel nach Anspruch 11, **dadurch gekennzeichnet, dass** das Oxidationsmittel Wasserstoffperoxid ist.

13. Verwendung einer Kombination aus
(a) 6,1 bis 25 Gewichtsprozent mindestens eines Fettalkoholes mit 14 bis 20 Kohlenstoffatomen,
(b) 1 bis 20 Gewichtsprozent mindestens eines Fettsäurealkanolamides und
(c) 1 bis 15 Gewichtsprozent mindestens eines Fettalkoholalkoxylates und/oder Fettsäurealkoxylates,
wobei das Gewichtsverhältnis von Fettalkohol (a) zu Alkanolamid (b) in der Farbträgermasse gleich 3:1 bis 1:2 und gleichzeitig das Verhältnis von Fettalkohol (a) zu Alkoxylat (c) gleich 4:1 bis 1:1,5 ist
und keine monomeren quaternären Ammoniumverbindungen sowie anionischen und kationischen Emulgatoren und Tensiden enthalten sind, zur Erzeugung eines Perlglanzeffektes in Farbträgermassen und Färbemitteln für Keratinfasern.

## Claims

1. A dye carrier mass comprising oxidative and/or non-oxidative dyes, **characterized in that** it is free from monomeric quaternary ammonium compounds as well as anionic and cationic emulsifiers and surfactants, and it contains a combination of
(a) 6.1 to 25% by weight of at least one fatty alcohol with 14 to 20 carbon atoms,
(b) 1 to 20% by weight of at least one fatty acid alkanolamide and
(c) 1 to 15% by weight of at least on fatty alcohol alkoxylate and/or fatty acid alkoxylate
wherein the weight ratio of fatty alcohol (a) to fatty acid alkanolamide (b) is 4:1 to 1:3 and at the same time the weight ratio of fatty alcohol (a) to alkoxylate (c) is 5:1 to 1:2.

2. The dye carrier mass according to Claim 1, **characterized in that** the fatty alcohol (a) and the fatty acid alkanolamide (b) are present in a weight ratio of 3:1 to 1:3 and at the same time the ratio of fatty alcohol (a) to alkoxylate (c) is 4:1 to 1:1.5.

3. The dye carrier mass according to Claim 1, **characterized in that** the fatty alcohol (a) and the fatty acid alkanolamide (b) are present in a weight ratio of 2.5:1 to 1:1 and at the same time the ratio of fatty alcohol (a) to alkoxylate (c) is 3:1 1 to 1:1.

4. The dye carrier mass according to any of Claims 1 to 3, **characterized in that** the fatty alcohol (a) is selected from cetyl alcohol, stearyl alcohol, myristyl alcohol, isooctyl alcohol, isotridecyl alcohol as well as mixtures of these compounds.

5. The dye carrier mass according to any of Claims 1 to 4, **characterized in that** the alkanoamide (b) is selected from N-acyl derivatives of monoethanolamine or diethanolamine as well as from the esters of monoethanolamine or diethanolamine.

6. The dye carrier mass according to any of Claims 1 to 5, **characterized in that** the alkoxylate (c) is selected from the group of fatty alcohol polyalkylene glycol ethers and fatty acid polyalkylene glycol esters with 8 to 30 carbon atoms in the fatty alcohol group or fatty acid group and 2 to 300 alkylene glycol units in the polyalkylene glycol group.

7. The dye carrier mass according to any of Claims 1 to 6, **characterized in that** the component (a) is contained in a total amount from 6.1 to 25% by weight.

8. The dye carrier mass according to any of Claims 1 to 7, **characterized in that** the component (b) is contained in a total amount from 1 to 20% by weight.

9. The dye carrier mass according to any of Claims 1 to 8, **characterized in that** the component (c) is contained in a total amount from 1 to 15% by weight.

10. The dye carrier mass according to any of Claims 1 to 9, **characterized in that** it contains, in addition to the compositions from component (c), additional further nonionic and/or amphoteric surfactants.

11. An agent for oxidative dying of hair, **characterized in that** it is obtained by mixing a dye carrier mass according to any of Claims 1 to 10 with an oxidizing agent.

12. The agent according to Claim 11, **characterized in that** the oxidizing agent is hydrogen peroxide.

13. Use of a combination containing
(a) 6.1 to 25% by weight of at least one fatty alcohol with 14 to 20 carbon atoms,
(b) 1 to 20% by weight of at least one fatty acid alkanolamide and
(c) 1 to 15% by weight of at least on fatty alcohol alkoxylate and/or fatty acid alkoxylate
wherein the weight ratio of fatty alcohol (a) to alkanolamide (b) in the dye carrier mass is 3:1 to 1:2 and at the same time the weight ratio of fatty alcohol (a) to alkoxylate (c) is 4:1 1 to 1:1.5
and no monomeric quaternary ammonium compounds as well as anionic and cationic emulsifiers and surfactants are contained,
to create a pearlescent effect in dye carrier masses and dyes for keratin fibers.

## Revendications

1. Masse de support de coloration contenant des colorants oxydatifs et/ou non oxydatifs, **caractérisée en ce qu'**elle est dépourvue de composés ammonium quaternaires monomères et d'émulsifiants et tensioactifs anioniques et cationiques, et une combinaison de 6,1 à 25 % en poids d'au moins un alcool gras comportant 14 à 20 atomes de carbone, 1 à 20 % en poids d'au moins un alcanolamide d'acide gras et 1 à 15 % en poids d'au moins un alcoxylat d'alcool gras et/ou un alcoxylat d'acide gras, le rapport en poids de l'alcool gras (a) à l'alcanolamide d'acide gras (b) étant de 4:1 à 1:3 et en même temps, le rapport en poids de l'alcool gras (a) à l'acoxylat (c) étant de 5:1 à 1:2.

2. Masse de support de coloration selon la revendication 1, **caractérisée en ce que** l'alcool gras (a) et l'alcanolamide d'acide gras (b) se situent en un rapport en poids de 3:1 à 1:2 et en même temps, le rapport de l'alcool gras (a) à l'alcoxylat (c) est de 4:1 à 1:1,5.

3. Masse de support de coloration selon la revendication 1, **caractérisée en ce que** l'alcool gras (a) et l'alcanolamide d'acide gras (b) se situent en un rapport en poids de 2,5:1 à 1:1 et en même temps, le rapport de l'alcool gras (a) à l'alcoxylat (c) est de 3:1 à 1:1.

4. Masse de support de coloration selon l'une des revendications 1 à 3, **caractérisée en ce que** l'alcool gras (a) est choisi parmi l'alcool de cétyle, l'alcool de stéaryle, l'alcool de myristyle, l'alcool d'isooctyle, l'alcool d'isotridécyle et les mélanges de ces composés.

5. Masse de support de coloration selon l'une des revendications 1 à 4, **caractérisée en ce que** l'alcanolamide (b) est choisi parmi les dérivés de N-acyle de monoéthanolamine ou diéthanolamine et les esters de monoéthanolamine ou diéthanolamine.

6. Masse de support de coloration selon l'une des revendications 1 à 5, **caractérisée en ce que** l'alcoxylat (c) est choisi dans le groupe des éthers d'alcools gras polyalkylène-glycol et les esters d'acide gras polyalkylène-glykol comportant 8 à 30 atomes de carbone dans le radical d'alcool gras ou le radical d'acide gras et 2 à 300 motifs alkylène-glykol dans le radical polyalkylène-glycol.

7. Masse de support de coloration selon l'une des revendications 1 à 6, **caractérisée en ce que** les composants (a) sont contenus en une quantité totale de 6,1 à 25 % en poids.

8. Masse de support de coloration selon l'une des revendications 1 à 7, **caractérisée en ce que** les composants (b) sont contenus en une quantité totale de 1 à 20 % en poids.

9. Masse de support de coloration selon l'une des revendications 1 à 8, **caractérisée en ce que** les composants (c) sont contenus en une quantité totale de 1 à 15 % en poids.

10. Masse de support de coloration selon l'une des revendications 1 à 9, **caractérisée en ce qu'**elle contient, outre les composés des composants (c), d'autres tensioactifs non ioniques et/ou amphotères.

11. Moyen de coloration oxydatif des cheveux, **caractérisé en ce qu'**il est obtenu par mélange d'une masse de support de coloration selon l'une des revendications 1 à 10, avec un agent d'oxydation.

12. Moyen selon la revendication 11, **caractérisé en ce que** l'agent d'oxydation est le peroxyde d'hydrogène.

13. Utilisation d'une combinaison de
(a) 6,1 à 25 % en poids d'au moins un alcool gras comportant 14 à 20 atomes de carbone,
(b) 1 à 20 % en poids d'au moins un alcanolamide d'acide gras et
(c) 1 à 15 % en poids d'au moins un alcoxylat d'alcool gras et/ou un alcoxylat d'acide gras,
le rapport en poids de l'alcool gras (a) à l'alcanolamide (b) dans la masse de support couleur étant de 3:1 à 1:2 et en même temps, le rapport de l'alcool gras (a) à l'alcoxylat (c) étant de 4:1 à 1:1,5
et aucun composé d'ammonium quaternaire monomère ni émulsifiant et tensioactifs anioniques et cationiques n'étant contenus, pour produire un effet nacré dans une masse de support de coloration et de colorants des fibres de kératine.
